# EUROPEAN PATENT APPLICATION

(11) **EP 4 691 474 A1**
(43) Date of publication of application: **11.02.2026**
(21) Application number: 24785427.6
(22) Date of filing: 11.03.2024
(51) Int. Cl.: A61K 31/7048, A61K 47/10, A61K 47/20, A61P 31/04

(54) **INJECTABLE PHARMACEUTICAL COMPOSITION COMPRISING TYLVALOSIN TARTRATE AND PRODUCTION METHOD**

(30) Priority: 05.04.2023 PE 0013712023
(71) Applicant: Agrovet Market S.A., San Luis, Lima, 15001 (PE)
(72) Inventor: CASTRO ROJAS, Elizabeth, Lima, 15001 (PE); MOLLEAPAZA AGUILAR, Erick Alfredo, Lima, 15001 (PE); DELGADO CRISPIN, Karen Melissa, Lima, 15001 (PE); CALDERON OJEDA, Jorge Umberto, Lima, 15001 (PE)
(74) Representative: JD&P Patent Attorneys Joanna Dargiewicz & Partners
(86) International application number: PCT/PE2024/050005
(87) International publication number: WO 2024/210760

(57) **Abstract**

The present invention is directed to a pharmaceutical formulation or pharmaceutical composition comprising Tylvalosin tartrate and a method of manufacturing or producing the same for injectable application and veterinary use, wherein the formulation or pharmaceutical composition is non-aqueous and contains adjuvants to stabilize the macrolide antibiotic such as Tylvalosin to be administered parenterally.

## Description

### TECHNICAL FIELD

The present invention is framed in the technical field of the pharmaceutical industry, mainly with the industry of pharmaceutical products for veterinary use.

### STATE OF THE ART

The development of pharmaceutical compositions or formulations comprising Tylvalosin where this active ingredient is stabilized, mainly for parenteral administration, is a current need for the treatment and prevention of respiratory and enteric diseases caused by microorganisms sensitive to Tylvalosin, such as chronic respiratory disease due to mycoplasmosis, necrotic enteritis, bacterial enteritis and infections by *Ornithobacterium rhinotracheale,* mycoplasmic pneumonia, ileitis, dysentery and porcine colitis.

In the field of patents, document WO2020/049570 is known. refers to the stability of a sustained release formulation and the effect that said stability has on the release profile of the active agent wherein using a combination of a poloxamer, an organic solvent and optionally a cellulose derivative in a sustained release formulation of an antimicrobial agent results in a stable injectable dispersion formulation, which has a consistent and reproducible release profile. Thus, there is provided a composition comprising a poorly soluble antimicrobial agent, at least one poloxamer, an organic solvent and a cellulose derivative that is at least partially soluble in organic solvents, and an aqueous medium, wherein said composition is injectable and the active ingredient is above 35% by weight or 40% by weight, wherein the active ingredient can be selected from florfenicol, lincomycin, tylosin, metronidazole, tilmicosin, spiramycin, erythromycin, tulathromycin, tiamulin, ampicillin, amoxicillin, clavulanate, penicillin, streptomycin, trimethoprim, sulfonamide, sulfamethoxazole, pleuromutilin, avilosin, tylvalosin, doxycycline and oxytetracycline in organic solvent such as dimethyl sulfoxide (DMSO) and propylene glycol.

It is also known from WO2021/183762 which teaches immunomodulatory compounds of formula (I) and stereoisomers thereof and pharmaceutically acceptable salts thereof wherein R0, R1, R2, R10, W, Ring C and n are as defined in the description, their stereoisomers and their pharmaceutically acceptable salts; and compositions comprising said compounds. The invention also includes the administration of additional agents such as macrolide ionophores (e.g., erythromycin, gamithromycin, tildipirosin, tilmicosin, tulathromycin, the M9 metabolite of tulathromycin, tylosin, tylvalosin, etc.) in solvents such as benzyl alcohol and antioxidants such as butyl hydroxy anisole.

On the other hand, patent document WO2016/112317 teaches a method for treating a disease caused by a filarial worm infection using a macrolide antibiotic, the method includes administering a therapeutically effective amount of a macrolide antibiotic to a subject suffering from a disease caused by a filarial worm infection. The macrolide antibiotic is tylosin A, a tylosin A analogue, a tylosin A derivative or a salt thereof. The macrolide antibiotic is tylosin tartrate (commercially available as Tylan^{®}) and in certain embodiments, the macrolide antibiotic is Tylvalosin tartrate (commercially available as Aivlosin^{®}) or tilmicosin phosphate (commercially available as Micotil^{®}). The composition may comprise glycols such as propylene glycol for injectable preparations and the active ingredient may be in a proportion of 50 to 90% by weight in liquid preparations.

Patent CN107485604 relates to an improved soluble powder of Tylvalosin tartrate which includes, by mass, 5 to 25 parts of Tylvalosin tartrate, 5 to 40 parts of cyclodextrin, 45 to 85 parts of a water-soluble carrier, 0.1 to 1 part of a sour taste conditioner, 0.1 to 1 part of a corrective and 0.05-0.5 part of an attractant. The soluble powder is prepared by sieving the components and mixing the components. The solubility (calculated based on Tylvalosin tartrate which is an effective component) of the soluble powder at room temperature may be 20000 ppm or higher, while the solubility of ordinary soluble powder of Tylvalosin tartrate is 10000 ppm. The solubility of Tylvalosin tartrate is obviously increased, and the improved Tylvalosin tartrate soluble powder has good stability, good palatability and high drug bioavailability.

Patent document CN107308119 teaches high water solubility Tylvalosin tartrate particles and a method of preparing the same. The high water solubility Tylvalosin tartrate particle comprises 5-30% Tylvalosin tartrate, 15-20% water soluble starch, 43-76.5% anhydrous glucose powder, 1-2% dimethyl sulfoxide 0.5-1% hydroxypropyl methylcellulose and 2-4% L-tartaric acid. The preparation method consists of step 1 comprising performing hot melting over dimethyl sulfoxide under a water bath at 40 °C, sieving Tylvalosin tartrate, water soluble starch, anhydrous glucose powder, dimethyl sulfoxide, hydroxypropyl methylcellulose and L-tartaric acid with 80 mesh sieve, and weighing according to formula for further use; a step 2, consisting of sequentially adding dimethyl sulfoxide, hydroxypropyl methylcellulose, water-soluble starch and L-tartaric acid in step 1 into hot water at 60 °C, and performing stirring for dissolution to obtain a mixture; step 3, mixing tylvalosin tartrate with anhydrous glucose powder in step 1 to obtain a mixture; and step 4, taking the mixture a from step 2 as an adhesive, performing granulation by using a vertical fluidized bed.

For its part, document CN107213470 refers to a soluble powder of Tylvalosin tartrate and a method for preparing it. The method comprises the steps of mixing hydroxypropyl-beta-cyclodextrin and water and stirring until dissolved to obtain a first liquid; mixing the first liquid, a crude drug of Tylvalosin tartrate and a pH regulator in a low-temperature water bath and stirring until a clear liquid is obtained; and spray-drying the clear liquid obtained through a spray-drying tower to obtain a powder. The prepared soluble powder of Tylvalosin tartrate is capable of masking the odor of the drug and is highly soluble and stable.

In this sense, there is still a need to provide a stable pharmaceutical composition or formulation of an antibiotic macrolide, mainly Tylvalosin tartrate, to be administered parenterally, which allows ensuring the dosage, treatment and prevention of respiratory and enteric diseases caused by microorganisms sensitive to Tylvalosin in infected animals.

### BRIEF DESCRIPTION OF THE INVENTION

The present invention is directed to a pharmaceutical formulation or pharmaceutical composition and a method of manufacturing or producing the same containing an injectable antibiotic for veterinary use comprising Tylvalosin tartrate, wherein the formulation or pharmaceutical composition is non-aqueous and contains adjuvants to stabilize macrolide antibiotics such as Tylvalosin to be administered parenterally.

Macrolide antimicrobials are primarily active against Gram-positive bacteria and mycoplasmas and show only limited activity against many Gram-negative bacteria. The main chemical characteristic common to all macrolides is the presence of a macrocyclic lactone ring. The macrolide antimicrobials used in veterinary medicine are tylosin, tilmicosin, and tylvalosin. These two are semisynthetic derivatives of tylosin. 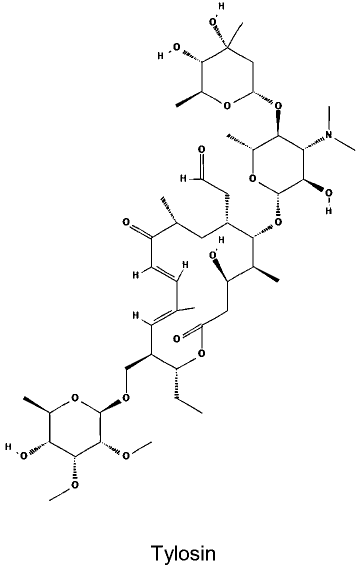 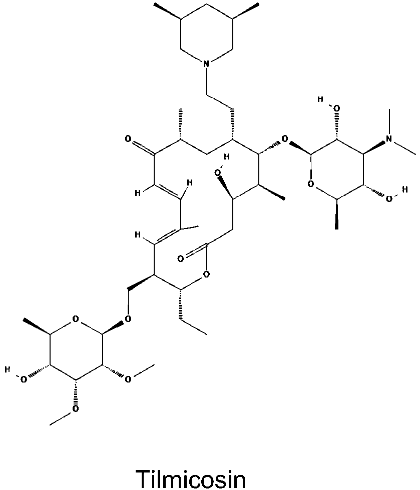 4A-O-De(2,6-dideoxy-3-C-methyl-a-L-ribo-hexopyranosyl)-20-deoxo-20-(3,5-dimethyl-1-piperidinyl)tylosin 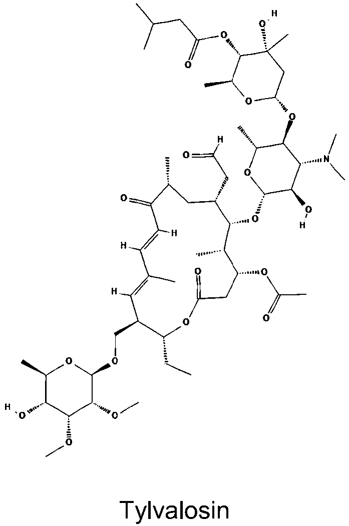 3-acetyl-4"-isovaleryl tylosin tartrate

### DETAILED DESCRIPTION OF THE INVENTION

In a first aspect, the present invention refers to a pharmaceutical composition or formulation for parenteral administration that stabilizes the macrolide antibiotic active ingredient, especially Tylvalosin, specifically Tylvalosin tartrate, wherein the composition or formulation comprises the active ingredient in a proportion between 5% and 20%, preferably between 10% and 15% and even more preferably 10% by weight of the total composition and pharmaceutically acceptable excipients.

Pharmaceutically acceptable excipients or components that may be in the pharmaceutical composition or formulation are an organic solvent, a preservative, antioxidants and a cosolvent or diluent.

The solvent may be an organic solvent such as dimethyl sulfoxide (DMSO) and may be in a proportion between 10% and 60% by weight of the final pharmaceutical formulation and more preferably 40%.

The preservative may be an aromatic alcohol, such as benzyl alcohol (C₆H₅CH₂OH) and may be in the pharmaceutical composition or formulation in a proportion between 1% and 10% by weight of the final formulation and more preferably 5%.

The antioxidant may be one or more synthetically produced antioxidants, such as butylhydroxyanisole (BHA)(C₁₁H₁₆O₂) and butylhydroxytoluene (BHT)(C₁₅H₂₄O) and may be in the pharmaceutical composition or formulation in a proportion between 0.0002 and 0.03% by weight of the final formulation and more preferably 0.01%.

The cosolvent or diluent may be a diol such as propylene glycol (C₃H₈O₂) and may be in the pharmaceutical composition or formulation in a proportion between 10% and 60% by weight of the final formulation and more preferably in sufficient quantity to bring to volume.

In a second aspect, the present invention relates to the method of producing an injectable pharmaceutical composition comprising Tylvalosin tartrate, wherein the method comprises the steps of:
a) Dissolve the antioxidant(s) in the preservative;
b) Disperse the active ingredient in the cosolvent or diluent;
c) Add the result from step b) to the result from step a) and mix until completely dissolved; and
d) Make up to 100% with the diluent.

In step a) the dissolution of the antioxidant(s) is carried out in a container of adequate capacity with mechanical stirring until complete dissolution, where the stirring is carried out for a time of between 5 to 20 minutes, preferably for 10 minutes.

In step b), the dispersion of the active ingredient in the cosolvent or diluent is done in another container with mechanical stirring for a time between 15 minutes and 60 minutes, preferably between 30 minutes and 45 minutes, even more preferably for a time of 45 minutes.

In step c) the mixing is carried out by stirring for a time between 10 minutes and 40 minutes, preferably between a time of 15 minutes to 30 minutes, preferably for a time of 20 minutes.

In step d) each fraction is completed to volume with the cosolvent by stirring until complete homogenization for a time between 5 minutes to 20 minutes, preferably between 10 minutes to 15 minutes.

Table 1 shows some of the formulations evaluated during the study, these were prepared following the same guidelines, a) the antioxidants are dissolved b) the macrolide active ingredient is dissolved, in this case Tylvalosin tartrate, c) the volume is leveled and d) the pH is adjusted within the established range, in this case between 6.0 and 7.0.

**Table 1. Qualitative and quantitative composition of pharmaceutical formulations of an injectable macrolide**

| | **Concentration in the formula (%)** | | | | |
|---|---|---|---|---|---|
| **Components** | **A** | **B** | **C** | **D** | **E** |
| Tylvalosin tartrate | 10.0 | 10.0 | 10.0 | 10.0 | 10.0 |
| Monothioglycerol | 0.5 | 0.5 | 0.5 | - | - |
| Benzyl alcohol | - | - | - | 5.0 | 5.0 |
| Butylhydroxytoluene | - | - | - | 0.01 | 0.01 |
| Butylhydroxyanisole | - | - | - | 0.01 | 0.01 |
| Methyl paraben | 0.18 | - | - | - | - |
| Propyl paraben | 0.02 | - | - | - | - |
| Hydrochloric acid | 0.67 | - | - | - | - |
| Phosphoric acid | - | - | - | 0.95 | - |
| Anhydrous citric acid | - | - | 3.0 | - | - |
| Dimethyl sulfoxide | - | q.s. 100.00 | - | 40.0 | 40.0 |
| Propylene glycol | q.s. 100.00 | - | q.s. 100.00 | q.s. 100.00 | q.s. 100.00 |

The different formulas were stored at 30 °C and 40 °C degrees, to determine if the formula remains stable under these conditions, for the study the samples were analyzed using the high performance liquid chromatography technique, the samples were analyzed at the beginning, at 7 days and at 6 months, the results are shown in table 2.

**Table 2. Results of the quantitative analysis of Tylvalosin in the formulations under study**

| **Formula** | **Temperature (°C)** | **Tylvalosin Concentration (%)** | **Evaluation time** |
|---|---|---|---|
| A | - | 83.8 | Start |
| | 30 | 81.4 | 7 days |
| | 40 | 79.3 | 7 days |
| B | - | 89.9 | Start |
| | 30 | 89.2 | 7 days |
| | 40 | 88.6 | 7 days |
| C | - | 37.5 | Start |
| | 30 | 4.4 | 7 days |
| | 40 | 0.0 | 7 days |
| D | - | 95.9 | Start |
| | 30 | 83.8 | 7 days |
| | 40 | 48.8 | 7 days |
| E | - | 102.1 | Start |
| | 30 | 99.6 | 6 months |
| | 40 | 101.7 | 6 months |

As can be seen, formulas A, B, C and D show a degradation greater than 10% with respect to the initial tylvalosin tartrate content, so the samples were reanalyzed after 7 days of storage at 30 °C and 40 °C to verify the degradation trend and if temperature is a crucial factor in this regard. In the case of formulas A and B, no significant correlation was found; however, in formula D, an impact of temperature on the formulation is observed, while formula C is shown to be the most unstable formula, having a degradation greater than 60% from the beginning and when stored at 30 °C and 40 °C the % of tylvalosin was 4 and 0% respectively. Formula E is the most stable from the initial analysis it shows that there is no degradation of the active ingredient during the manufacturing process. After 6 months of storing the sample at 30 and 40 degrees the degradation of the active ingredient is minimal, the variation obtained is contemplated within the standard deviation of 2% expected by the analytical methodology.

## Claims

1. An injectable pharmaceutical composition comprising Tylvalosin tartrate, **characterized in that** it comprises Tylvalosin tartrate in a proportion between 5% and 20% by weight of the total composition and also comprises an organic solvent, a preservative, antioxidants and a cosolvent or diluent.

2. The injectable pharmaceutical composition comprising Tylvalosin tartrate according to claim 1, **characterized in that** the organic solvent is dimethyl sulfoxide in a proportion between 10% and 60% by weight of the final composition.

3. The injectable pharmaceutical composition comprising Tylvalosin tartrate according to claim 1, **characterized in that** the antioxidant(s) are butylhydroxyanisole (BHA)(C₁₁H₁₆O₂) and butylhydroxytoluene (BHT) (C₁₅H₂₄O) in a proportion between 0.0002 and 0.03% by weight of the final composition.

4. The injectable pharmaceutical composition comprising Tylvalosin tartrate according to claim 1, **characterized in that** the cosolvent or diluent is propylene glycol (C₃H₈O₂) in a proportion between 10% and 60% by weight of the final composition.

5. The injectable pharmaceutical composition comprising Tylvalosin tartrate according to claim 1, **characterized in that** the preservative is benzyl alcohol (C₆H₅CH₂OH) in a proportion between 1% and 10% by weight of the final composition.

6. A method of producing an injectable pharmaceutical composition comprising Tylvalosin tartrate, wherein the method comprises the steps of:
a) Dissolve the antioxidant(s) in the preservative;
b) Disperse the active ingredient in the cosolvent or diluent;
c) Add the result from step b) to the result from step a) and mix until completely dissolved; and
d) Make up to 100% with the diluent.
